# EUROPEAN PATENT SPECIFICATION

(11) **EP 1 246 140 B1**
(45) Date of publication and mention of the grant of the patent: **25.01.2006**
(21) Application number: 02006987.8
(22) Date of filing: 27.03.2002
(51) Int. Cl.: G07F 11/42, G07F 11/26, G07F 11/16

(54) **Transfusion bottle feed apparatus**
Zuführvorrichtung für Infusions- und Transfusionsflaschen
Dispositif d'alimentation de flacons d'infusion ou de transfusion

(30) Priority: 30.03.2001 JP 2001101972
(43) Date of publication of application: 02.10.2002
(73) Proprietor: Yuyama Mfg. Co., Ltd., Toyonaka-shi, Osaka 561-0841 (JP)
(72) Inventor: Yuyama, Shoji, Toyonaka-shi, Osaka 561-0841 (JP); Kodama, Tsuyoshi, Toyonaka-shi, Osaka 561-0841 (JP); Kitagawa, Reiji, Toyonaka-shi, Osaka 561-0841 (JP)
(74) Representative: Selting, Günther

(56) References cited:
- WO-A-00/34925
- WO-A-01/68484
- FR-A- 2 699 310
- US-A- 5 048 719
- PATENT ABSTRACTS OF JAPAN vol. 015, no. 253 (M-1129), 27 June 1991 (1991-06-27) & JP 03 083701 A (SEIBU ELECTRIC & MACH CO LTD), 9 April 1991 (1991-04-09)
- PATENT ABSTRACTS OF JAPAN vol. 011, no. 039 (M-559), 5 February 1987 (1987-02-05) & JP 61 206708 A (MOTODA ELECTRONICS CO LTD), 13 September 1986 (1986-09-13)

## Description

### BACKGROUND OF THE INVENTION

The present invention relates to a transfusion bottle feed apparatus.

Conventionally, there have been proposed various kinds of transfusion bottle apparatuses in which a plurality of transfusion bottles are grouped by their kinds and stored in the apparatus body by putting the plurality of transfusion bottles on storage shelves so that the transfusion bottles can be collected in accordance with a prescription data and fed to a patient.

In the conventional transfusion bottle apparatuses, the transfusion bottles having a capacity of 100 ml more or less to be frequently used occupy the most part of storage shelves. Thus, many kind of the transfusion bottles can not be stored and therefore a plurality of same apparatuses must be installed. Further, since many kinds of transfusion bottles exist in admixture, a mechanism for conveying each transfusion bottle to a collection container is complicated, whereby it takes long time to collect the transfusion bottles.

Moreover, in the conventional transfusion bottle apparatuses, since the transfusion bottle is gripped and dropped into the collection container from a fixed height, when a small transfusion bottle having a capacity of 100 ml more or less is dropped, it damages the transfusion bottles previously collected in the collection container.

WO 00/34925 to which the preamble of claim 1 refers discloses a storage and dispensing system for storing and dispensing pre-packaged pharmaceutical products and other products. The system includes a cabinet, an infeed and an outfeed conveyor. The infeed conveyor receives products that are to be stored in the cabinet. The outfeed conveyor receives products that are to be dispensed from the cabinet. A plurality of product conveyors is mounted within the cabinet and each configured for holding at least one product. A transporter is movable within the cabinet for transporting products between the product conveyors and the infeed and outfeed conveyors for storing products in or dispensing products from the cabinet. The single products are dispensed separately apart from each other and not in batches or groups.

### SUMMARY OF THE INVENTION

It is an object of the present invention to provide a transfusion bottle feed apparatus which can rapidly collect transfusion bottles with a simple conveying mechanism.

It is an another object of the present invention to provide a transfusion bottle feed apparatus which never damage the transfusion bottles.

The subject matter of the invention is defined by claim 1.

In order to achieve the aforementioned objects, the present invention provides a transfusion bottle feed apparatus in which a plurality of transfusion bottles are grouped by their kinds and stored in an apparatus body so that the transfusion bottles can be fed to a patient in accordance with a prescription data.

Plural stages of storage shelves are provided on which packaging boxes with the upper surface opened are put and stored, each of the packaging boxes contains same kind of the transfusion bottles, the packaging boxes in which same kind of the transfusion bottles are contained being put on the storage shelves in an up-and-down direction.

A first conveyor is provided on each of the storage shelves for conveying the packaging box of the transfusion bottle stored on the storage shelves.

A lifter is provided which can move upward and downward and can stop at the same levels as that of the storage shelves and at the level of a receiving table.

A second conveyor is provided on the lifter for receiving and conveying the packaging box of the transfusion bottle conveyed from the storage shelves by the first conveyor.

The receiving table is positioned at a position lower than the lowermost stage ot the plural stages of storage shelves.

A third conveyor is provided on the receiving table for receiving and conveying the packaging box of the transfusion bottle conveyed from the lifter by the second conveyor mechanism.

A grip mechanism is provided for gripping the transfusion bottle in the packaging box positioned on the receiving table, conveying to a collection container which is conveyed on a conveyor path inside the apparatus body, releasing the transfusion bottle above the collection container to contain it therein.

In the present invention having above construction, the lifter is moved upward until the storage shelf on which the packaging box that contains the transfusion bottles corresponding to the prescription is stored. Then, the first conveyor mechanism of the storage shelf and the second conveyor mechanism of the lifter are driven to convey the packaging box of the transfusion bottle from the storage shelf to the lifter. When the lifter is moved downward to the lowermost position, the second conveyor mechanism of the lifter and the third conveyor mechanism of the receiving table are driven to convey the packaging box of the transfusion bottle from the lifter to the receiving table. Subsequently, the grip mechanism is driven to grip the transfusion bottle in the packaging box on the receiving table and convey it to the collection container. Then, the transfusion bottle is released above the collection container and contained therein. Thus, the transfusion bottle is collected with the simple mechanism operation and rapidly fed to a patient.

Preferably, the grip mechanism is provided with a tilting mechanism for tilting the transfusion bottle gripped by the grip mechanism; and the gripping mechanism moves downward until the transfusion bottle tilted by the tilting mechanism comes into contact with the bottom of the collection container and releases the transfusion bottle to move in the horizontal direction. Thus, the transfusion bottle falls down with the inclined state, reducing the impact and preventing the transfusion bottles below from being damaged.

Preferably, the receiving table is slidable toward an opening of the apparatus body; and at the position where the receiving table is slid to the opening of the body apparatus, the third conveyor mechanism discharges the empty packaging box on the receiving table through the opening of the apparatus body. Thus, the packaging box with no transfusion bottle can be easily taken out. In this case, providing a lever for kicking the rear portion of the empty packaging box upward allows the packaging box to be easily discharged through the opening of the apparatus body.

### BRIEF DESCRIPTION OF THE DRAWINGS

Other objects and features of the present invention will become clear from the following detail description with reference to the accompanying drawings in which:
Fig. 1 is a front view showing a transfusion bottle feed apparatus according to the present invention;
Fig. 2 is a side view of Fig. 1;
Fig. 3 is a sectional view taken along III - III line of Fig. 1;
Fig. 4 is a sectional view taken along IV - IV line of Fig. 1; and
Fig. 5 is a side view showing a discharge operation of empty packaging box.

### DETAILED DESCRIPTION OF THE PREFERRED EMBODIMENTS

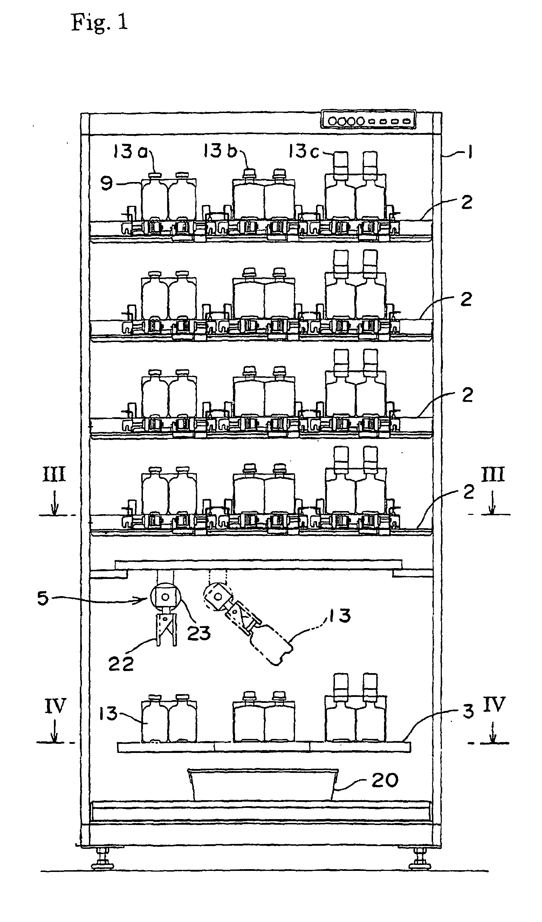

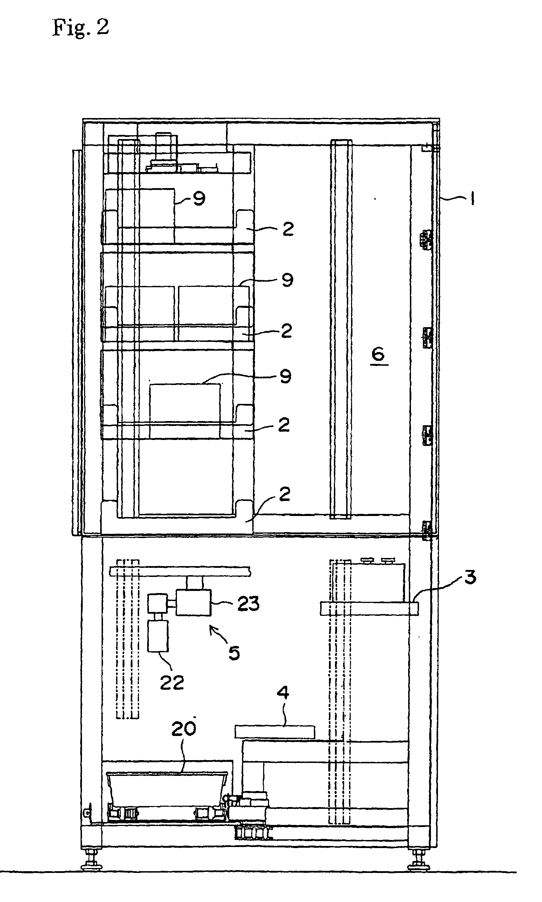

Figs. 1 and 2 show a transfusion bottle feed apparatus according to the present invention. In the transfusion bottle feed apparatus, an apparatus body 1 having a rectangular frame is provided with a plural stages of storage shelves 2, a lifter 3, a receiving table 4 and a grip mechanism 5.

The storage shelves 2 are positioned at almost upper part of the apparatus body 1. Behind the storage shelves 2 is provided a lifting space 6 for the lifter 3 which will be explained hereinafter. At each storage section (three sections are shown in the figure) of the respective storage shelf 2, a plurality of conveyor rollers 8 (first conveyor mechanism) which are driven to rotate by a motor 7 are provided so that a packaging box 9 put on the plurality of conveyor rollers 8 can be conveyed in a back-and-forth direction of the apparatus body 1. Further, at both the front side of the conveyor roller 8 positioned at the foremost position and the rear side of the conveyor roller 8 positioned at the backmost position, stoppers 10, 11 are provided in order to prevent the packaging box 9 from dropping off. The rear stopper 11 is arranged so as to be horizontally laid by a motor 12.

On each storage shelf 2, the packaging boxes 9 in each of which the same kind of the transfusion bottles 13 are contained are put and stored with the upper surface opened. In an up-and-down direction of the storage shelves 2, the packaging boxes 9 in which the same kind of the transfusion bottles 13 are contained are stored. Namely, in Fig. 1, the packaging boxes 9 in which "A" of the transfusion bottles 13a are contained are stored in the left column; the packaging boxes 9 in which "B" of the transfusion bottles 13b are contained are stored in the middle column; and the packaging boxes 9 in which "C" of the transfusion bottles 13c are contained are stored in the right column.

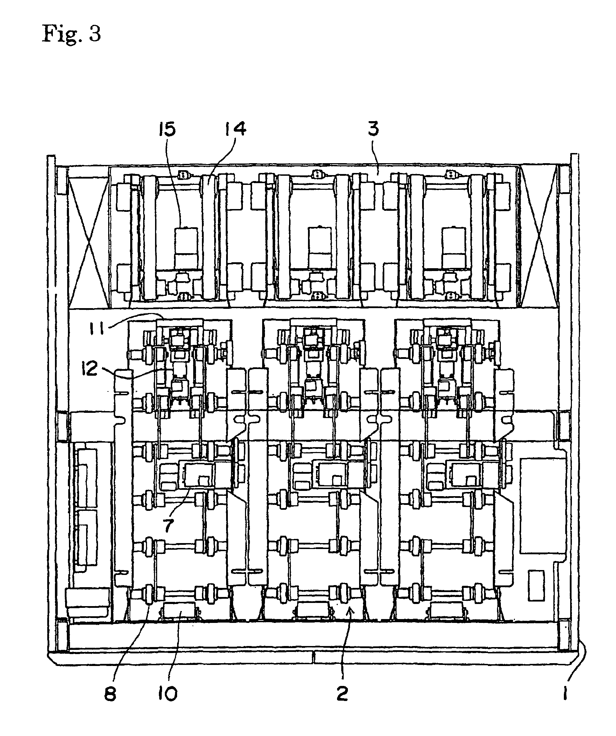

The lifter 3 is arranged so that it can move upward and downward in the lifting space 6 behind the storage shelves 2 and can stop at the same levels as that of the storage shelves 2. On the lifter 3, as shown in Fig. 3, a plurality of conveyor belts 14 (second conveyor mechanism) the number of which corresponds to that of the storage sections of each storage shelf 2 are provided. Each of the conveyor belts 14 is driven to rotate by a motor 15 so that the packaging box 9 put on the conveyor belt 14 can be conveyed in the same direction as that of the conveyor rollers 8 of the storage shelf 2.

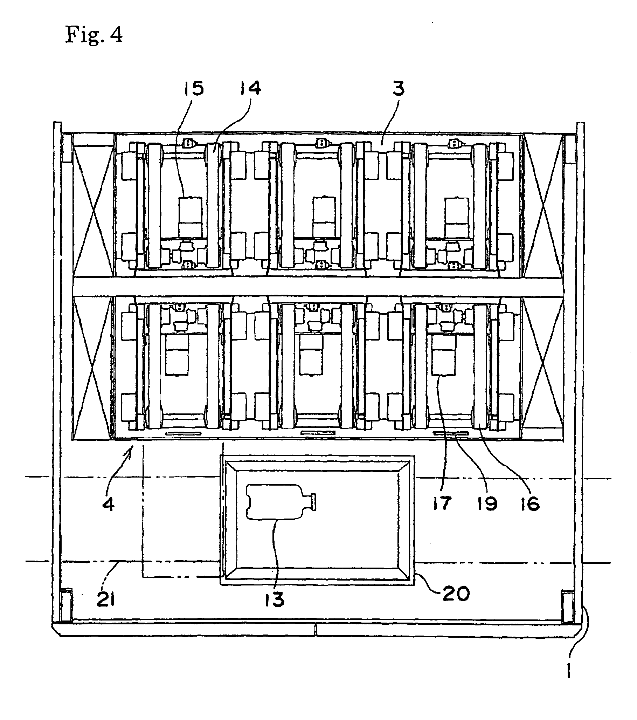

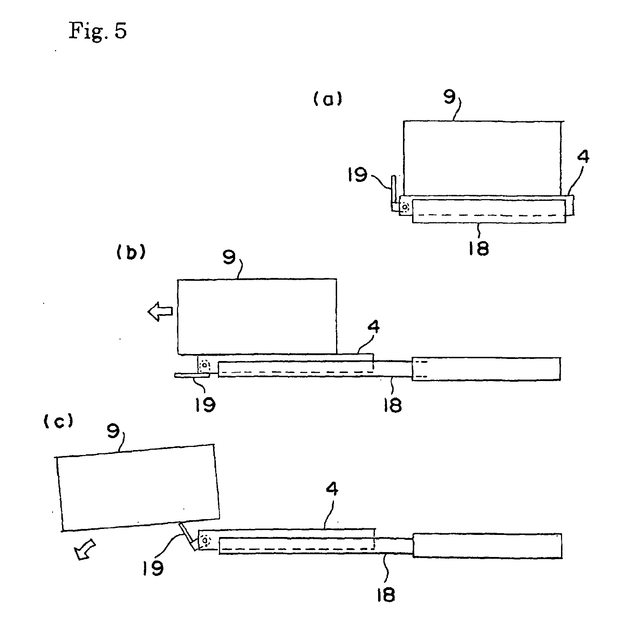

The receiving table 4, as shown in Fig. 4, is disposed at the same level as the lifter 3 moved downward to the lowermost position and at the front side of the lifter 3. On the receiving table 4, a plurality of conveyor belts 16 (third conveyor mechanism) are provided in the same manner as the conveyor belts 14 of the lifter 3. Each of the conveyor belts 16 is driven to rotate by a motor 17 so that the packaging box 9 put on the conveyor belt 16 can be conveyed in the same direction as that of the conveyor belts 14 of the lifter 3. Further, the receiving table 4, as shown in Fig. 5, is slidably moved forward by a motor (not shown) on guide rails 18 toward an opening formed on the apparatus body 1. At the front portion of the receiving table 4, there are provided levers 19 each of which also serves as a stopper and each of which is arranged so as to be horizontally laid by a motor not shown.

At the front side of the receiving table 4, as shown in Fig. 4, there is disposed a conveyor path 21 which passes through the apparatus body 1 and on which a collection container 20 is conveyed in a left-and-right direction.

The grip mechanism 5, as shown in Figs. 1 and 2, comprises a grip arm 22 which can grip and release the mouth portion of the transfusion bottle 13 from upward, a tilting mechanism 23 which can tilt the grip arm 22 by about 60 degrees, and a moving mechanism not shown which can move the grip arm 22 upward and downward and also move the grip arm 22 in both a back-and-forth direction and a left-and-right direction.

Operation of the transfusion bottle feed apparatus having aforementioned construction will be explained hereinafter.

When a prescription data is inputted to a control unit not shown of the transfusion bottle feed apparatus, the collection container 20 is conveyed on the conveyor path 21 and positioned in the transfusion bottle feed apparatus. Then, the lifter 3 is moved upward to the storage shelf 2 on which the packaging boxes 9 that contain the transfusion bottles 13 corresponding to the prescription are stored. The conveyor rollers 8 of the storage shelf 2 and the conveyor belts 14 of the lifter 3 are driven to convey the packaging box 9 of the transfusion bottle 13 from the storage shelf 2 to the lifter 3. Consequently, the lifter 3 is moved downward to the lowermost position. The conveyor belts 14 of the lifter 3 and the conveyor belts 16 of the receiving table 4 are driven to convey the packaging box 9 of the transfusion bottle 13 from the lifter 3 to the receiving table 4. Then, the grip mechanism 5 is driven to grip the transfusion bottle 13 within the packaging box 9 on the receiving table 4. The transfusion bottle 13 is moved to the collection container 20 and released above the collection container 20, whereby the transfusion bottle 13 is contained in the collection container 20. Thus, with the simple operation of the mechanisms, the transfusion bottle 13 is collected and rapidly fed.

When the transfusion bottle 13 is contained in the collection container 20, as shown in two-dots chain line in Fig. 1, the grip arm 22 is tilted by about 60 degrees by the tilting mechanism 23 of the grip mechanism 5 in a state that the grip arm 22 grips the transfusion bottle 13. With this state kept, the grip arm 22 is moved downward until the transfusion bottle 13 comes into contact with the bottom of the collection container 20. Then, the grip arm 22 releases the transfusion bottle 13 and retreats in a horizontal direction. Thus, the transfusion bottle 13 falls down with the inclined state, reducing the impact and preventing the transfusion bottles below from being damaged.

When the transfusion bottle 13 in the packaging box 9 becomes shortage, as shown in Figs. 5(a) and 5(b), the receiving table 4 is slidably moved forward on the guide rails 18 toward the opening of the apparatus body 1, where the lever 19 is laid and the transfer belts 16 are driven to discharge the empty packaging box 9 on the receiving table 4 through the opening of the apparatus body 1. At this time, as shown in Fig. 5(c), when the lever 19 is rose up, the rear portion of the empty packaging box 9 is kicked upward, allowing the empty packaging box 9 to be easily discharged through the opening.

## Claims

1. A transfusion bottle feed apparatus in which a plurality of transfusion bottles (13) are grouped by their kinds and stored in an apparatus body (1) so that the transfusion bottles (13) can be fed to a patient in accordance with a prescription data, comprising
plural stages of storage shelves (2) on which packaging boxes (9) with the upper surface opened are put and stored, each of the packaging boxes (9) contain same kind of the transfusion bottles (13), the packaging boxes (9) in which same kind of the transfusion bottles (13) are contained being put on the storage shelves (2) in an up-and-down direction;
a first conveyor (8) provided on each of the storage shelves (2) for conveying the packaging box (9) of the transfusion bottle (13) stored on the storage shelves (2);
a receiving table (4);
a lifter (3) which can move upward and downward and can stop at the same levels as that of the storage shelves (2) and at the level of the receiving table 4;
a second conveyor (14) provided on the lifter (3) for receiving and conveying the packaging box (9) of the transfusion bottle (13) conveyed from the storage shelves (2) by the first conveyor (8);
a third conveyor (16) provided on the receiving table (4) for receiving and conveying the packaging box (9) of the transfusion bottle (13) conveyed from the lifter (3) by the second conveyor (14)
**characterized in that**
said receiving table (4) is positioned at a position lower than the lowermost stage of the plural stages of storage shelves (2);
a grip mechanism (5) is provided for gripping the transfusion bottle (13) in the packaging box (9) positioned on the receiving table (4), conveying it to a collection container (20) which is conveyed on a conveyor path (21) inside the apparatus body (1), releasing the transfusion bottle (13) above the collection container (20) to contain it therein.

2. The transfusion bottle feed apparatus as in claim 1, wherein the grip mechanism (5) is provided with a tilting mechanism (23) for tilting the transfusion bottle (13) gripped by the grip mechanism (5); and the gripping mechanism (5) moves downward until the transfusion bottle (13) tilted by the tilting mechanism (23) comes into contact with the bottom of the collection container (20) and releases the transfusion bottle (13) to move in the horizontal direction.

3. The transfusion bottle feed apparatus as in claim 1, wherein the receiving table (4) is slidable toward an opening of the apparatus body (1); and at the position where the receiving table (4) is slid to the opening of the apparatus body (1), the third conveyor (16) discharges the empty packaging box (9) on the receiving table (4) through the opening of the apparatus body (1).

4. The transfusion bottle feed apparatus as in claim 3, further comprising a lever (19) for kicking the rear portion of the empty packaging box (9) upward.

5. The transfusion bottle feed apparatus as in claim 1, wherein the grip mechanism (5) has a grip arm (22) which can grip the transfusion bottle (13) and can move upward and downward and also move in both a back-and-forth direction and a left-and-right direction, whereby the grip arm (22) can grip the transfusion bottle (13) in the packaging box (9) resting on a conveyor belt (16) of the receiving table (4) to move the transfusion bottle (13) to the collection container (20) which is conveyed on the conveyor path (21) and positioned inside the apparatus.

## Patentansprüche

1. Infusions- oder Transfusionsflaschenzuführvorrichtung, bei der mehrere Infusions- oder Transfusionsflaschen (13) nach ihrem Typ gruppiert und in einem Vorrichtungskörper (1) gelagert sind, so daß die Infusions- oder Transfusionsflaschen (13) einem Patienten entsprechend Verschreibungsdaten zugeführt werden können, mit
mehrere Stufe von Regalfächern (2), auf denen Verpackungsbehälter (9) mit offener Oberseite abgestellt und gelagert sind, wobei jede der Verpackungsschachteln (9) die gleiche Art von Infusions- oder Transfusionsflaschen (13) enthält und die Verpackungsbehälter (9), welche die gleiche Art von Infusions- oder Transfusionsflaschen (13) enthalten, in von oben nach unten verlaufender Richtung in den Regalfächern (2) angeordnet sind;
einer in jedem Regalfach (2) vorgesehenen ersten Fördereinrichtung (8) zum Fördern der Verpackungsbehälter (9) der Infusions- oder Transfusionsflaschen (13), die auf den Regalfächern (2) gelagert sind;
einem Aufnahmetisch (4);
einer Hebeeinrichtung (3), die auf- und abwärts bewegbar ist und auf der Höhe der jeweiligen Regalfächer (2) und auf der Höhe des Aufnahmetischs (4) anhalten kann;
einer an der Hebeeinrichtung (3) vorgesehenen zweiten Fördereinrichtung (14) zum Aufnehmen und Fördern des Verpackungsbehälters (9) der Infusions- oder Transfusionsflasche (13), die durch die erste Fördereinrichtung (8) von den Regalfächern (2) zugeführt wurde;
einer am Aufnahmetisch (4) vorgesehenen dritten Fördereinrichtung (16) zum Aufnehmen und Fördern des Verpackungsbehälters (9) der Infusions- oder Transfusionsflasche (13), die durch die zweite Fördereinrichtung (14) von der Hebeeinrichtung (3) zugeführt wurde;
**dadurch gekennzeichnet, daß**
der Aufnahmetisch (4) an einer Position angeordnet ist, die tiefer als die unterste Stufe mehreren Stufen von Regalfächern (2) liegt;
ein Greifmechanismus (5) zum Greifen der auf dem Aufnahmetisch (4) angeordneten Infusions- oder Transfusionsflasche (13) in dem Verpakkungsbehälter (9), Fördern derselben zu einem Sammelbehälter (20), der auf einem Förderweg (21) im Inneren des Vorrichtungsgehäuses (1) transportiert wird, Freigeben der Infusions- oder Transfusionsflasche (13) über dem Sammelbehälter (20), um sie darin aufzunehmen.

2. Infusions- oder Transfusionsflaschenzuführvorrichtung nach Anspruch 1, bei der der Greifmechanismus (5) mit einem Kippmechanismus (23) zum Kippen der von dem Greifmechanismus (5) gegriffenen Infusions- oder Transfusionsflasche (13) versehen ist, und der Greifmechanismus (5) sich abwärts bewegt, bis die von dem Kippmechanismus (23) gekippte Infusions- oder Transfusionsflasche (13) den Boden des Sammelbehälters (20) berührt, und die Infusions- oder Transfusionsflasche (13) zur Bewegung in horizontaler Richtung freigibt.

3. Infusions- oder Transfusionsflaschenzuführvorrichtung nach Anspruch 1, bei welcher der Aufnahmetisch (4) in Richtung einer Öffnung des Vorrichtungskörpers (1) gleitend verschiebbar ist, und die dritte Fördereinrichtung (16) an der Position, in welche der Aufnahmetisch (4) zu der Öffnung des Vorrichtungskörpers (1) gleitend verschoben ist, den leeren Verpackungsbehälter (9) durch die Öffnung des Vorrichtungskörpers (1) auf den Aufnahmetisch (4) ausgibt.

4. Infusions- oder Transfusionsflaschenzuführvorrichtung nach Anspruch 3, ferner mit einem Hebel (19) zum Stoßen des hinteren Teils des leeren Verpackungsbehälters (9) nach oben.

5. Infusions- oder Transfusionsflaschenzuführvorrichtung nach Anspruch 1, bei welcher der Greifmechanismus (5) einen Greifarm (22) aufweist, der die Infusions- oder Transfusionsflasche (13) greifen und sich auf und ab sowie sowohl vor und zurück, als auch nach links und rechts bewegen kann, wodurch der Greifarm (22) die Infusions- oder Transfusionsflasche (13) in dem Verpackungsbehälter (9), die auf einem Förderband (16) des Aufnahmetischs (4) ruht, greifen kann, um die Infusions- oder Transfusionsflasche (13) zu dem Sammelbehälter (20) zu bewegen, der auf der Förderbahn (21) transportiert wird und im Inneren der Vorrichtung angeordnet ist.

## Revendications

1. Dispositif d'alimentation de flacon de transfusion dans lequel une pluralité de flacons de transfusion (13) est groupée par type et stockée dans un corps de dispositif (1) de telle sorte que les flacons de transfusion (13) peuvent être délivrés à un patient en fonction de données de prescription, comprenant :
plusieurs niveaux d'étagères de stockage (2) sur lesquelles des boîtes de conditionnement (9) dont la surface supérieure est ouverte sont placées et stockées, chacune des boîtes de conditionnement (9) contient le même type de flacons de transfusion (13), les boîtes de conditionnement (9), dans lesquelles le même type de flacons de transfusion (13) est contenu, étant placées sur les étagères de stockage (2) suivant une direction verticale ;
un premier transporteur (8) agencé sur chacune des étagères de stockage (2) afin d'assurer le transfert de la boîte de conditionnement (9) du flacon de transfusion (13) stocké sur les étagères de stockage (2) ;
une table de réception (4) ;
un élévateur (3) qui peut se déplacer vers le haut et vers le bas et peut s'arrêter aux mêmes niveaux que ceux des étagères de stockage (2) et au niveau de la table de réception (4) ;
un deuxième transporteur (14) agencé sur l'élévateur (3) afin de recevoir et de transférer la boîte de conditionnement (9) du flacon de transfusion (13) transférée à partir des étagères de stockage (2) par le premier transporteur (8) ;
un troisième transporteur (16) agencé sur la table de réception (4) afin de recevoir et de transférer la boîte de conditionnement (9) du flacon de transfusion (13) transférée à partir de l'élévateur (3) par le deuxième transporteur (14)
**caractérisé en ce que** :
ladite table de transporteur (4) est positionnée à un emplacement plus bas que l'étage le plus bas de la pluralité de niveaux d'étagères de stockage (2) ;
un mécanisme de saisie (5) est agencé de manière à saisir le flacon de transfusion (13) dans la boîte de conditionnement (9) positionnée sur la table de réception (4), à le transférer vers un conteneur de collecte (20) qui est transféré sur un trajet de transporteur (21) à l'intérieur du corps du dispositif (1), libérant le flacon de transfusion (13) au-dessus du conteneur de collecte (20) afin de le retenir à l'intérieur.

2. Dispositif d'alimentation de flacon de transfusion selon la revendication 1, dans lequel le mécanisme de saisie (5) comporte un mécanisme de basculement (23) destiné à faire basculer le flacon de transfusion (13) saisi par le mécanisme de saisie (5) ; et le mécanisme de saisie (5) se déplace vers le bas jusqu'à ce que le flacon de transfusion (13), basculé par le mécanisme de basculement (23), arrive en contact avec le fond du conteneur de collecte (20) et libère le flacon de transfusion (13) pour se déplacer dans la direction horizontale.

3. Dispositif d'alimentation de flacon de transfusion selon la revendication 1, dans lequel la table de réception (4) peut coulisser vers une ouverture du corps de dispositif (1), et à l'emplacement auquel la table de réception (4) est glissée vers l'ouverture du corps de dispositif (1), le troisième transporteur (16) décharge la boîte de conditionnement vide (9) sur la table de réception (4) à travers l'ouverture du corps de dispositif (1).

4. Dispositif d'alimentation de flacon de transfusion selon la revendication 3, comprenant, en outre, un levier (19) afin de faire basculer la partie arrière de la boîte de conditionnement (9) vide vers le haut.

5. Dispositif d'alimentation de flacon de transfusion selon la revendication 1, dans lequel le mécanisme de saisie (5) comporte un bras de saisie (22) qui peut saisir le flacon de transfusion (13) et peut se déplacer vers le haut et vers le bas ainsi que se déplacer à la fois vers l'avant et vers l'arrière et vers la gauche et vers la droite, de telle sorte que le bras de saisie (22) peut saisir le flacon de transfusion (13) dans la boîte de conditionnement (9) reposant sur une bande de transporteur (16) de la table de réception (4) afin de déplacer le flacon de transfusion (13) vers le conteneur de collecte (20) qui est transféré sur le trajet de transporteur (21) et positionné à l'intérieur du dispositif.
